# EUROPEAN PATENT APPLICATION

(11) **EP 4 786 044 A1**
(43) Date of publication of application: **05.08.2026**
(21) Application number: 25155675.9
(22) Date of filing: 03.02.2025
(51) Int. Cl.: B01J 20/22, B01D 53/047, B01D 53/26, B01J 19/18, B01J 20/28, C07C 29/76

(54) **COMPOUND AND METHOD FOR SEPARATING AN ALCOHOL/WATER MIXTURE**

(71) Applicant: Technische Universität Dresden Körperschaft des öffentlichen Rechts, 01069 Dresden (DE)
(72) Inventor: Papadopoulos, Konstantinos, 01127 Dresden (DE); Bon, Volodymyr, 01159 Dresden (DE); Kaskel, Stefan, 01069 Dresden (DE)
(74) Representative: Riechelmann & Carlsohn Patentanwälte PartG mbB

(57) **Abstract**

The invention relates to a water-free phase of a (glycyl-L-histidinato)copper(II) compound. The water-free phase of the (glycyl-L-histidinato)copper(II) compound has the following symmetry and crystal structure: tetragonal, space group P4₃2₁2, a=b=11.0012(2) Å, c=18.8529(7) Å, α=β=γ=90° and V=2281.72(13) Å³. The temperature of the crystal structure determination was 283.15 K.

## Description

The invention relates to a compound suitable for separating a mixture containing an alcohol and water and a method for separating a mixture containing an alcohol and water. Furthermore, it relates to a use of an adsorbent and a device for separating a mixture containing an alcohol and water.

Short-chain alcohols such as methanol and ethanol are industrially relevant solvents and fuels. When prepared from water and carbon dioxide using electric energy they are also referred to as e-fuels.

Short-chain alcohols of general formula CₙH₂ₙ₊₁OH in general can be mixed with water without limitation. They are often prepared by means of fermentation methods to obtain diluted aqueous solutions. In industry, often use is made of a distillation method to separate the alcohol from water. By means of the distillation method alcohol is separated from alcohol/water mixtures. However, the distillation methods are energy-intensive. In case of ethanol an azeotropic mixture of 95.6% by mass of ethanol and 4.4% by mass of water is formed during distillation. Pure ethanol can only be obtained by chemical dehydration using desiccants such as P₂O₅ or a chemical reaction, for example a reaction with tetraethoxysilane.

The common way to separate alcohol and water is a distillation method at elevated temperatures. The method is energy-intensive since the alcohol/water mixtures have to be heated to the boiling temperature of the alcohol. To separate methanol/water mixtures no alternative methods have been described so far (L. M. Vane, Review: Membrane Materials for the Removal of Water from Industrial Solvents by Pervaporation and Vapor Permeation, Journal of chemical technology and biotechnology (Oxford, Oxfordshire: 1986), 2019, 94, 343-365). Therefore, methods for separating alcohol/water mixtures are needed that require less energy input.

The problem of the invention is to eliminate the drawbacks according to the prior art. Particularly, a method for separating a mixture shall be provided which contains an alcohol and water and requires less energy compared to separation by means of distillation.

The mentioned problem is solved by the features of claims 1, 3, 10, and 12. Suitable developments of the inventions result from the features of the dependent claims.

According to the invention a water-free phase of a (glycyl-L-histidinato)copper(II) compound is provided. The water-free phase of the (glycyl-L-histidinato)copper(II) compound can have the following symmetry and crystal structure: tetragonal, space group P4₃2₁2, a=b=11.0012(2) Å, c=18.8529(7) Å, α=β=γ=90° and V=2281.72(13) Å³. The temperature of the crystal structure determination was 283.15 K. Said compound is a water-free phase of the (glycyl-L-histidinato)copper(II) compound. This water-free phase of the (glycyl-L-histidinato)copper(II) compound in the following is also referred to as water-free α' phase of the (glycyl-L-histidinato)copper(II) compound. The water-free α' phase of the (glycyl-L-histidinato)copper(II) compound has no water molecules. In particular, it has no water molecules in its structure. Thus, the water-free α' phase of the (glycyl-L-histidinato)copper(II) compound is an anhydrous phase of the (glycyl-L-histidinato)copper(II) compound. The water-free phase of a (glycyl-L-histidinato)copper(II) compound has open pores that permit selective adsorption of water from a mixture containing an aliphatic alcohol and water.

J. F. Blount et al. (Crystallographic Studies of Metal-Peptide Complexes. IV. (Glycyl-L-histidinato)copper(II) Sesquihydrate, Acta Cryst. (1967). 22, 396) have described a (glycyl-L-histidinato)copper(II) compound which is a (glycyl-L-histidinato)copper(II) sesquihydrate. The (glycyl-L-histidinato)copper(II) sesquihydrate is a water-containing α phase of the (glycyl-L-histidinato)copper(II) compound. J. F. Blount et al. have given the following symmetry and lattice constant for the (glycyl-L-histidinato)copper(II) sesquihydrate: tetragonal, space group P4₃2₁2 with a=b=11.24(2) Å, c=17.84(4) Å, α=β=γ=90° and V=2253.86 Å³, wherein the temperature of the crystal structure determination of J. F. Blount et al. has not been given. The inventors of the present invention have found the following symmetry and lattice constant for the (glycyl-L-histidinato)copper(II) sesquihydrate: tetragonal, space group P4₃2₁2 with a=b=11.2298(1) Å, c=17.4197(2) Å, α=β=γ=90° and V=2196.77 Å³ (measured at T = 100 K). The (glycyl-L-histidinato)copper(II) sesquihydrate described by J. F. Blount et al. and the (glycyl-L-histidinato)copper(II) sesquihydrate investigated by the inventors have the same composition, both are water-containing, and both are S isomers. The inventors of the present invention have found that the water-containing α phase of the (glycyl-L-histidinato)copper(II) compound described by J. F. Blount et al. is not suitable for separating water and alcohol.

However, the inventors have found that a further phase of the (glycyl-L-histidinato)copper(II) compound does exist. This further phase is the water-free α' phase of the (glycyl-L-histidinato)copper(II) compound as described above. This phase contains no water molecules in the structure. The water-free α' phase of the (glycyl-L-histidinato)copper(II) compound has the following symmetry and crystal structure: tetragonal, space group P4₃2₁2, a=b=11.0012(2) Å, c=18.8529(7) Å, α=β=γ=90° and V=2281.72(13) Å³. The temperature of the crystal structure determination was 283.15 K. Said compound is a water-free α' phase of the (glycyl-L-histidinato)copper(II) compound. It is not known from the prior art.

The water-free phase of the (glycyl-L-histidinato)copper(II) compound provided according to the invention is a crystalline solid. It is a coordination polymer. The water-free phase of the (glycyl-L-histidinato)copper(II) compound is a phase of the (glycyl-L-histidinato)copper(II) compound and, thus, a (glycyl-L-histidinato)copper(II) compound for itself.

Carbonell, C. et al. (Femtolitre chemistry assisted by microfluidic pen lithography. Nat. Commun. 4:2173 (doi: 10.1038/ncomms3173 (2013)) have described the crystal structure of a water-containing β phase of a (glycyl-L-histidinato)copper(II) compound. The compound is published in the CSD data base under CCDC 912870. The β phase has the following symmetry and crystal structure: orthorhombic, P2₁2₁2₁, a=6.991(5) Å, b=10.778(5) Å, c=15.575(5) Å, α=β=γ=90° and V=1173.561 Å³. The inventors of the present invention have found that said β phase is not suitable for separating water and alcohol. Moreover, it contains water in its structure.

The inventors have found that the water-free α' phase of the (glycyl-L-histidinato)copper(II) compound can be used for separating water and alcohol while the (glycyl-L-histidinato)copper(II) sesquihydrate described by J. F. Blount et al. is not suitable for this purpose. The α phase of the (glycyl-L-histidinato)copper(II) compound and the α' phase of the (glycyl-L-histidinato)copper(II) compound are different phases even though the difference in the lattice constants is relatively small, they differ primarily in the presence or absence of water in the structure. Both, the (glycyl-L-histidinato)copper(II) sesquihydrate described by J. F. Blount et al. and the β phase described by C. Carbonell are water-containing phases and each contain symmetry-independent water molecules in their structures with the ratio of lattice water to copper-coordinated water being 1:2. The water-free α' phase of the (glycyl-L-histidinato)copper(II) compound prepared by the inventors contains no water.

The α phase of the (glycyl-L-histidinato)copper(II) compound, the α' phase of the (glycyl-L-histidinato)copper(II) compound and the β phase of the (glycyl-L-histidinato)copper(II) compound are S isomers.

A method for preparing (glycyl-L-histidinato)copper(II) sesquihydrate is known from J. F. Blount et al., Crystallographic Studies of Metal-Peptide Complexes. (Glycyl-L-histidinato)copper(II) Sesquihydrate, Acta Cryst. (1967). 22, 396. (Glycyl-L-histidinato)copper(II) sesquihydrate can be used to prepare the water-free (glycyl-L-histidinato)copper(II) compound. The water-free α' phase of the (glycyl-L-histidinato)copper(II) compound can be obtained from the (glycyl-L-histidinato)copper(II) sesquihydrate by dehydration. In the dehydration of the (glycyl-L-histidinato)copper(II) sesquihydrate pores are formed that permit selective adsorption of water from a mixture containing an aliphatic alcohol and water.

The inventors have found that the water-free α' phase of the (glycyl-L-histidinato)copper(II) compound has a pore size that permits selective adsorption of water from a mixture containing an aliphatic alcohol and water. Here, only one of the two components is adsorbed, namely the water. The pore size of the water-free α' phase of the (glycyl-L-histidinato)copper(II) compound is suited for the adsorption of water, but too small for the adsorption of an alcohol. The water-free α' phase of the (glycyl-L-histidinato)copper(II) compound is an open-pore material. Studies of the inventors have shown that the water-free α' phase of the (glycyl-L-histidinato)copper(II) compound can adsorb up to 6.5 mmol of water per gram of the water-free (glycyl-L-histidinato)copper(II) compound. Here, no alcohol or only a very small amount of an alcohol is adsorbed. This particularly applies to methanol and/or ethanol.

According to the invention further provided is a method for separating a mixture containing an aliphatic alcohol and water using an adsorbent to adsorb water , wherein the adsorbent is the water-free phase of the (glycyl-L-histidinato)copper(II) compound as described above. Preferably, the water-free phase of the (glycyl-L-histidinato)copper(II) compound is the water-free α' phase of a (glycyl-L-histidinato)copper(II) compound as described above.

The method according to the invention is based on the adsorption of water contained in the mixture on the adsorbent. The aliphatic alcohol is not adsorbed by the adsorbent. The separating effect of the method according to the invention is based on this effect. The method according to the invention requires less energy compared to separation by means of distillation.

The aliphatic alcohol can contain one or more hydroxyl groups. Preferably, the aliphatic alcohol comprises one, two or three hydroxyl groups. An aliphatic alcohol can be a diol or a triol. The aliphatic alcohol can be a compound of general formula R¹-OH, wherein R¹ is selected from the group consisting of a substituted or unsubstituted alkyl group having 1 to 12 carbon atoms, a substituted or unsubstituted alkenyl group having 2 to 12 carbon atoms, and a substituted or unsubstituted alkynyl group having 2 to 12 carbon atoms. Preferably, R¹ is an unsubstituted alkyl group having 1 to 12 carbon atoms, preferably an unsubstituted alkyl group having 1 to 9 carbon atoms, more preferably an unsubstituted alkyl group having 1 to 6 carbon atoms, and particularly preferred an unsubstituted alkyl group having 1 to 3 carbon atoms. For example, the aliphatic alcohol can be selected from the group consisting of methanol, ethanol, 1-propanol, 2-propanol, 1-butanol, 2-methyl-1-propanol, 2-butanol, and 2-methyl-2-propanol. Preferably, the aliphatic alcohol is selected from the group consisting of methanol, ethanol, 1-propanol, 2-propanol, ethane-1,2-diol, and propane-1,2,3-triol. Preferably, the aliphatic alcohol is selected from the group consisting of methanol, ethanol, 1-propanol, 2-propanol, 1-butanol, 2-methyl-1-propanol, 2-butanol, and 2-methyl-2-propanol. More preferably, the aliphatic alcohol is selected from the group consisting of methanol, ethanol, 1-propanol and 2-propanol with the aliphatic alcohol still more preferably being methanol or ethanol and particularly preferred methanol. The aliphatic alcohol is also referred to as "alcohol."

More preferably, the (glycyl-L-histidinato)copper(II) compound is a water-free α' phase of the (glycyl-L-histidinato)copper(II) compound.

The mixture containing an aliphatic alcohol and water may be a mixture containing only alcohol and water. The mixture can consist of alcohol and water. The mixture containing an aliphatic alcohol and water is also referred to as "mixture".

The method according to the invention can be carried out as a continuous or discontinuous method. The method according to the invention can comprise a regeneration of the adsorbent loaded with water. For example, water can be removed from the pores of the adsorbent by subjecting the adsorbent to vacuum at 80°C.

The adsorbent provided can be used to separate alcohol and water in a manner known per se. For example, the mixture can be led over a membrane having the (glycyl-L-histidinato)copper(II) compound. In one example, the separation provided according to the invention can be performed by means of pressure swing adsorption, preferably by vacuum swing adsorption. The pressure swing adsorption can comprise the following steps:
(a) adsorption of water on the adsorbent at a pressure in a range of from 100 kPa to 300 kPa; and
(b) desorption of water from the adsorbent at a pressure in a range of from 1 Pa to 100 Pa.

Step (a) is for separating the water from the mixture, wherein an anhydrous aliphatic alcohol or a mixture containing a small amount of water is obtained as the mixture supplied by the supply line. The adsorbent is loaded with water, thereby obtaining a loaded adsorbent.

Step (b) is for the regeneration of the loaded adsorbent. Separation of the water takes place by its desorption from the adsorbent. Here, an unloaded adsorbent is obtained which can be used again in step (a).

In another example the separation provided in accordance with the invention can be effected by means of pervaporation. Here, the mixture is provided as a liquid on one side of a membrane and a vacuum is applied on the other side. In this way, the water contained in the mixture is adsorbed on the membrane. Subsequently, the water permeates through the membrane.

The method according to the invention can be carried out as a continuous or discontinuous method.

The method according to the invention permits adsorptive separation of an aliphatic alcohol from water. The method according to the invention can be carried out at room temperature. For example, it can be carried out at a temperature of 15 to 25°C. Unless otherwise stated, the method according to the invention can be carried out at ambient pressure, for example 101.325 kPa.

According to the invention the use of a water-free phase of a (glycyl-L-histidinato)copper(II) compound as an adsorbent for adsorption of water is provided. In particular, the water-free phase of a (glycyl-L-histidinato)copper(II) compound can be used for the adsorptive separation of an aliphatic alcohol from water.

Preferably, the water-free phase of a (glycyl-L-histidinato)copper(II) compound is a water-free α' phase of the (glycyl-L-histidinato)copper(II) compound. The water-free α' phase of (glycyl-L-histidinato)copper(II) compound has the following symmetry and crystal structure: tetragonal, space group P4₃2₁2, a=b=11.0012(2) Å, c=18.8529(7) Å, α=β=γ=90° and V=2281.72(13) Å³. The temperature of the crystal structure determination was 283.15 K. The water-free α' phase of the (glycyl-L-histidinato)copper(II) compound can be used as a molecular sieve.

Further details on the use according to the invention have already been described in context with the compound and the method according to the invention. Reference is made to said description.

According to the invention there is further provided a device for separating a mixture containing an aliphatic alcohol and water using a water-free phase of a (glycyl-L-histidinato)copper(II) compound as an adsorbent for the adsorption of water. The device has a reactor containing the adsorbent or a membrane containing the adsorbent. Preferably, the (glycyl-L-histidinato)copper(II) compound is a water-free α' phase of the (glycyl-L-histidinato)copper(II) compound. The water-free α' phase of the (glycyl-L-histidinato)copper(II) compound has the following symmetry and crystal structure: tetragonal, space group P4₃2₁2, a=b=11.0012(2) Å, c=18.8529(7) Å, α=β=γ=90° and V=2281.72(13) Å³. The temperature of the crystal structure determination was 283.15 K. Further details of the water-free phase of the (glycyl-L-histidinato)copper(II) compound are described above.

It may be provided that the reactor is a fixed-bed reactor. The reactor may be a column. For the preparation of the column it may be provided that pellets of the (glycyl-L-histidinato)copper(II) compound and a binder are prepared and filled into the housing of the column. The binder may be methylcellulose, for example.

It may be provided that the device is a membrane to which the mixture of the (glycyl-L-histidinato)copper(II) compound and a polymer is applied. The membrane can be free-standing or on a support. The support can be flat or a tube. For example, the support can be a ceramic. For example, the polymer can be polyvinylidene fluoride (PVDF). For example, the mixture can be a mixture of 50% by weight of the (glycyl-L-histidinato)copper(II) compound and 50% by weight of polyvinylidene fluoride (PVDF).

The device can have a supply line via which the mixture containing the aliphatic alcohol and water is led into the reactor or to the membrane. The mixture is a feed mixture. The device can further have a discharge line through which the anhydrous aliphatic alcohol or a mixture containing a smaller amount of water as the mixture supplied by the supply line is led out of the reactor or away from the membrane. The obtained anhydrous aliphatic alcohol can be a concentrate. Further details of the membrane reactor and the membrane technology are disclosed in Diary Processing Handbook, English edition, third edition, revision 1, ISBN 978-9176111321, Section 6.4 "Membrane technology".

Further details on the device according to the invention have already been described in context with the compound and the method according to the invention. Reference is made to said description.

The term "alkyl", unless otherwise indicated, especially refers to a saturated aliphatic hydrocarbon group with a branched or unbranched carbon chain having 1 to 12 carbon atoms, preferably 1 to 8 carbon atoms, and particularly preferred 1 to 6 carbon atoms. Examples of alkyl groups comprise but are not limited to methyl, ethyl, propyl, isopropyl, isobutyl, *sec*-butyl, *tert*-butyl, pentyl, *n*-hexyl, octyl, dodecyl, and the like. The alkyl group can optionally be substituted with one or more substituents, wherein each substituent independently is hydroxy, alkyl, alkoxy, halogen, haloalkyl, -COOR (wherein R is hydrogen or alkyl), -CN, -N₃, -NO, -NO₂, -SR (wherein R is hydrogen or alkyl), amino, monoalkylamino, or dialkylamino, unless specifically indicated otherwise.

The term "alkenyl", unless otherwise indicated, especially refers to an unsaturated aliphatic hydrocarbon group with a branched or unbranched carbon chain having 2 to 12 carbon atoms, preferably 2 to 8 carbon atoms, and particularly preferred 2 to 6 carbon atoms and having at least one olefinic double bond and more preferably one single double bond. Examples of alkenyl groups comprise but are not limited to vinyl, allyl, methallyl, 1,1-dimethylallyl, propenyl, butenyl, pentadienyl, hexenyl, octenyl, and the like. An allyl group is preferred. The alkenyl group can optionally be substituted with one or more substituents, wherein each substituent independently is hydroxy, alkyl, alkoxy, halogen, haloalkyl, -COOR (wherein R is hydrogen or alkyl), -CN, -N₃, -NO, -NO₂, -SR (wherein R is hydrogen or alkyl), amino, monoalkylamino, or dialkylamino, unless specifically indicated otherwise.

The term "alkynyl", unless otherwise indicated, especially refers to an unsaturated aliphatic hydrocarbon group with a branched or unbranched carbon chain having 2 to 12 carbon atoms, preferably 2 to 8 carbon atoms, and particularly preferred 2 to 6 carbon atoms and having at least one olefinic triple bond and more preferably one single triple bond. Examples of alkynyl groups comprise but are not limited to acetylenyl, propargyl, n-but-2-yn-1-yl, and the like. A propargyl group is preferred. The alkynyl group can optionally be substituted with one or more substituents, wherein each substituent independently is hydroxy, alkyl, alkoxy, halogen, haloalkyl, -COOR (wherein R is hydrogen or alkyl) -CN, -N₃, -NO, -NO₂, -SR (wherein R is hydrogen or alkyl), amino, monoalkylamino, or dialkylamino, unless specifically indicated otherwise.

The term "alkoxy", unless otherwise indicated, especially refers to a group of formula -OR, wherein R is an alkyl group, as defined herein. Examples of alkoxy components comprise but are not limited to methoxy, ethoxy, isopropoxy, and the like. The alkoxy group can optionally be substituted with one or more substituents, wherein each substituent independently is hydroxy, alkyl, alkoxy, halogen, haloalkyl, amino, monoalkylamino, or dialkylamino, unless specifically indicated otherwise.

The term "halogen", unless otherwise indicated, especially refers to fluorine, chlorine, bromine, or iodine.

In the following, the invention is explained in more detail with the help of examples not intended to limit the invention with respect to the drawings. Here,
- Fig. 1: shows a representation of a crystal structure of the water-free α' phase of the (glycyl-L-histidinato)copper(II) compound determined by means of single crystal X-ray diffraction analysis;
- Fig. 2: shows a representation of the coordination environment of the Cu(II) metal sites of the crystal structure shown in Fig. 1; and
- Fig. 3: shows a graph illustrating the physisorption isotherms of water and methanol on the water-free α' phase of the (glycyl-L-histidinato)copper(II) compound.

### Example 1

### Determination of the crystal structure of the water-free α' phase of the (glycyl-L-histidinato)copper(II) compound

The crystal structure of the water-free α' phase of the (glycyl-L-histidinato)copper(II) compound was determined at 283.15 K by means of single crystal X-ray diffraction analysis as follows. The procedure was as follows: experimental data were acquired at room temperature (rt) with a Rigaku XtaLAB Synergy-S diffractometer. The instrument is equipped with a microfocus X-ray source (Mo-Kα radiation, λ=71.073 pm) and a hybrid photon counting detector (Eiger2 R 1M CdTe, Dectris). The raw data were processed with the CrysAlisPro software, and an automated empiric absorption correction was performed. The structures were solved with the SHELXT¹ software and refined by full matrix least square analysis (SHELXL)^{2,3} using the OLEX2⁴ program package. Non-hydrogen atoms were anisotropic improved, and hydrogen atoms were set on ideal geometries and improved with fixed isotropic displacement parameters in the "Riding" model. The graphic material of the structure was drawn up with the Mercury software.

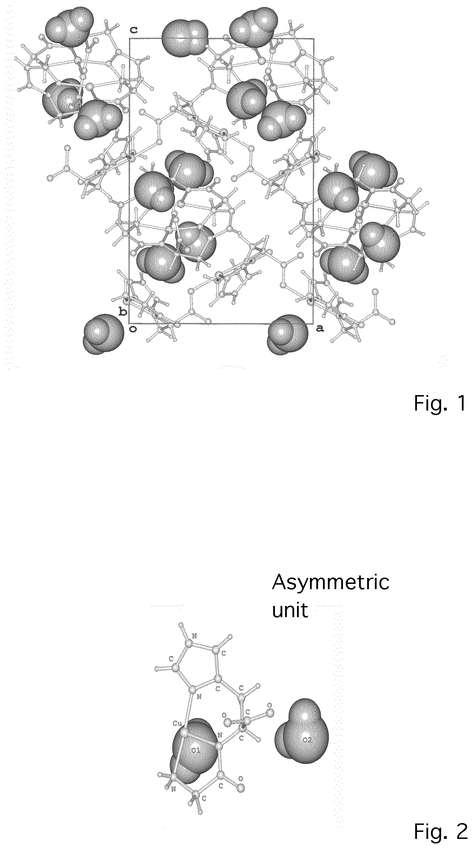

Fig. 1 shows the determined crystal structure of the water-free α' phase of the (glycyl-L-histidinato)copper(II) compound. Fig. 2 shows the coordination environment of the Cu(II) metal sites of the crystal structure of the water-free α' phase of the (glycyl-L-histidinato)copper(II) compound. Here, the asymmetric unit is shown. The following crystal structure was found for the water-free α' phase of the (glycyl-L-histidinato)copper(II) compound: tetragonal, space group P4₃2₁2 with a=b=11.0012(2) Å, c=18.8529(7) Å, α=β=γ=90°, V=2281.72(13) Å³ and Flack parameter (x) = -0.006(11). The temperature of the crystal structure determination was 283.15 K.

The Flack parameter enables a distinction to be made between enantiomers. The Flack parameter is determined by means of single crystal X-ray diffraction analysis. To distinct between enantiomers also the anomalous scattering phenomenon, which can also be determined by means of single crystal X-ray diffraction, and the circular dichroism can be used. Usually, phases can be differentiated by means of X-ray powder diffraction data. However, with chiral compounds distinction between Rand S isomers only on the basis of the X-ray powder diffraction data is not possible. The water-free α' phase of the (glycyl-L-histidinato)copper(II) compound described in example 1 is the S isomer.

The coordinates of the atoms for the water-free α' phase of the (glycyl-L-histidinato)copper(II) compound are represented in table 1.

**Table 1 Atom coordinates for the crystal structure of the water-free α' phase of the (glycyl-L-histidinato)copper(II) compound**

| Atom | x | y | z | U(eq) [Å²] |
|---|---|---|---|---|
| Cu1 | 0.49308(6) | 0.22348(6) | 0.59318(3) | 0.0314(2) |
| 013 | 0.7532(4) | 0.0262(4) | 0.4971(3) | 0.0497(14) |
| 015 | 0.3388(3) | 0.2509(4) | 0.6426(2) | 0.0383(12) |
| 016 | 0.4243(4) | 0.1649(5) | 0.7360(2) | 0.0550(16) |
| N2 | 0.5757(5) | 0.5703(5) | 0.6496(3) | 0.0420(17) |
| N5 | 0.5341(4) | 0.3957(4) | 0.6004(3) | 0.0357(16) |
| N9 | 0.6335(4) | 0.1843(4) | 0.5339(2) | 0.0300(12) |
| N12 | 0.4716(4) | 0.0432(4) | 0.5952(3) | 0.0420(17) |
| C3 | 0.1479(5) | -0.0612(6) | 0.6598(3) | 0.0397(17) |
| C4 | 0.1210(5) | 0.0474(5) | 0.6906(3) | 0.0317(17) |
| C6 | 0.5088(6) | 0.4701(5) | 0.6535(3) | 0.0417(17) |
| C7 | 0.1614(5) | 0.1039(5) | 0.7581(3) | 0.0347(17) |
| C8 | 0.2199(5) | 0.2293(5) | 0.7482(3) | 0.0293(16) |
| C10 | 0.6636(5) | 0.0704(5) | 0.5294(3) | 0.0363(17) |
| C11 | 0.5839(6) | -0.0174(6) | 0.5698(4) | 0.0480(19) |
| C14 | 0.3372(5) | 0.2160(5) | 0.7063(3) | 0.0317(17) |

The specification "U(eq)" designates the equivalent isotropic atomic temperature factor.

### Example 2

### Preparation of the water-free α' phase of the (glycyl-L-histidinato)copper(II) compound

Compound (glycyl-L-histidinato)copper(II) sesquihydrate was synthesized in accordance with the experimental procedure described in the article of J. F. Blount et al., Crystallographic Studies of Metal-Peptide Complexes. IV. (Glycyl-L-histidinato)copper(II) Sesquihydrate, Acta Cryst. (1967). 22, 396. (Glycyl-L-histidinato)copper(II) sesquihydrate was used as the starting material in the following synthesis of the water-free α' phase of the (glycyl-L-histidinato)copper(II) compound.

100 mg of (glycyl-L-histidinato)copper(II) sesquihydrate were stored in a Schlenk tube. The Schlenk tube was connected to a vacuum line, wherein the dynamic vacuum is at least 10 Pa. The Schlenk tube was baked out at 80°C for at least one hour. Subsequently, the Schlenk tube was purged with dry argon and the product, i.e. the water-free α' phase of the (glycyl-L-histidinato)copper(II) compound, was funneled into an anhydrous glove box.

### Example 3

### Determination of physisorption isotherms of water and methanol

The physisorption isotherms of water and methanol of the water-free α' phase of the (glycyl-L-histidinato)copper(II) compound were determined at 298 K. This was done as follows: The isotherms were recorded by means of volumetric methods with the commercial automated BELSORP max adsorption instrument. Typically, 15 to 20 mg of the water-free α' phase of the (glycyl-L-histidinato)copper(II) compound were baked out over 12 hours in the dynamic vacuum at 80°C in the measuring cell and subsequently attached to the apparatus. Distilled water or anhydrous methanol were used for the measurements. The following conditions were chosen for the adsorption equilibrium: pressure loss of 0.5% within 450 s for the entire pressure range.

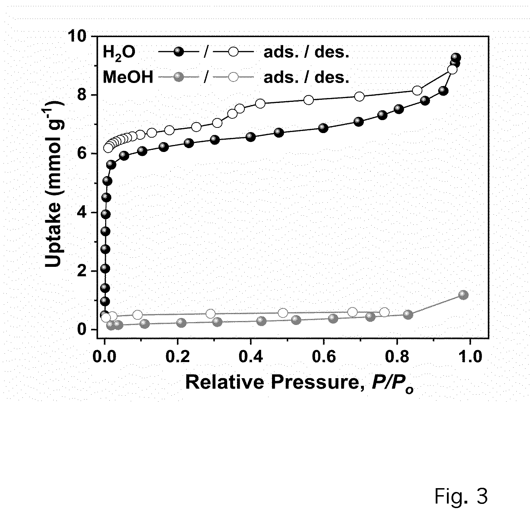

The graph shown in Fig. 3 as the abscissa shows the relative pressure P/Pₒ, wherein P indicates the absolute value of the adsorption equilibration pressure and Pₒ the saturation vapor pressure. The ordinate quantitatively shows the adsorbed amount of methanol or water in mmol, per mass of the adsorbent in g. The specification "H₂O ads" indicates the water adsorbed on the adsorbent, the specification "MeOH ads" indicates the methanol adsorbed on the adsorbent. The specification "H₂O des" indicates the water desorbed by the adsorbent. It can be seen that hardly any methanol is adsorbed. Hysteresis between adsorption and desorption curves results from the slow adsorption kinetics.

### Example 4

### Determination of the crystal structure of the water-containing α phase of the (glycyl-L-histidinato)copper(II) compound

The crystal structure of the water-containing α phase of the (glycyl-L-histidinato)copper(II) compound was determined by means of single crystal X-ray diffraction analysis, as described in example 1, except that the determination was performed at 100 K. The following crystal structure was found for the water-containing α phase of (glycyl-L-histidinato)copper(II) compound: tetragonal, space group P4₃2₁2 with a=b=11.2298(1) Å, c=17.4197(2) Å, α=β=γ=90°, V=2196.77 Å³. The temperature of the crystal structure determination was 100 K. Said water-containing α phase of the (glycyl-L-histidinato)copper(II) compound is the (glycyl-L-histidinato)copper(II) sesquihydrate described by J. F. Blount et al.

### Literature

(1) Sheldrick, G. M. SHELXT - Integrated Space-Group and Crystal-Structure Determination. Acta Crystallogr. 2015, A71 (1), 3-8. https://doi.org/10.1107/S2053273314026370.
(2) Sheldrick, G. M. A Short History of SHELX. Acta Crystallogr. 2008, A64 (1), 112-122. https://doi.org/10.1107/S0108767307043930.
(3) Sheldrick, G. M. Crystal Structure Refinement with SHELXL. Acta Crystallogr. 2015, C71 (1), 3-8. https://doi.org/10.1107/S2053229614024218.
(4) Dolomanov, O. V.; Bourhis, L. J.; Gildea, R. J.; Howard, J. a. K.; Puschmann, H. OLEX2: A Complete Structure Solution, Refinement and Analysis Program. J. Appl. Crystallogr. 2009, 42 (2), 339-341. https://doi.org/10.1107/S0021889808042726.

The work leading to this invention has received funding from BMBF under grant agreement n° BMBF 05K22OD1 and 05K22OD2.

## Claims

1. A water-free phase of a (glycyl-L-histidinato)copper(II) compound.

2. The water-free phase of the (glycyl-L-histidinato)copper(II) compound according to claim 1, **characterized in that** it has the following symmetry and crystal structure: tetragonal, space group P4₃2₁2, a=b=11.0012(2) Å, c=18.8529(7) Å, α=β=γ=90° and V=2281.72(13) Å³.

3. A method for separating a mixture containing an aliphatic alcohol and water using an adsorbent for the adsorption of water, wherein the adsorbent is a water-free phase of a (glycyl-L-histidinato)copper(II) compound.

4. The method according to claim 3, **characterized in that** the water-free phase of the (glycyl-L-histidinato)copper(II) compound has the following symmetry and crystal structure: tetragonal, space group P4₃2₁2, a=b=11.0012(2) Å, c=18.8529(7) Å, α=β=γ=90° and V=2281.72(13) Å³.

5. The method according to claim 3 or claim 4, **characterized in that** the aliphatic alcohol is a compound of general formula R¹-OH, wherein R¹ is selected from the group consisting of a substituted or unsubstituted alkyl group having 1 to 12 carbon atoms, a substituted or unsubstituted alkenyl group having 2 to 12 carbon atoms, and a substituted or unsubstituted alkynyl group having 2 to 12 carbon atoms.

6. The method according to any of claims 3 to 5, **characterized in that** the aliphatic alcohol is selected from the group consisting of methanol, ethanol, 1-propanol, 2-propanol, 1-butanol, 2-methyl-1-propanol, 2-butanol, 2-methyl-2-propanol, ethane-1,2-diol, and propane-1,2,3-triol.

7. The method according to any of claims 3 to 6, **characterized in that** the mixture is led over a membrane having water-free phase of the (glycyl-L-histidinato)copper(II) compound.

8. The method according to any of claims 3 to 7, **characterized in that** the separation is performed by means of pressure swing adsorption.

9. The method according to any of claims 3 to 8, **characterized in that** the separation is performed by means of pervaporation.

10. Use of a water-free phase of a (glycyl-L-histidinato)copper(II) compound as an adsorbent for the adsorption of water.

11. The use according to claim 10, **characterized in that** the water-free phase of the (glycyl-L-histidinato)copper(II) compound has the following symmetry and crystal structure: tetragonal, space group P4₃2₁2 with a=b=11.0012(2) Å, c=18.8529(7) Å, α=β=γ=90° and V=2281.72(13) Å³.

12. A device for separating a mixture containing an aliphatic alcohol and water using a water-free phase of a (glycyl-L-histidinato)copper(II) compound as an adsorbent for the adsorption of water, wherein the device has a reactor containing the adsorbent or a membrane containing the adsorbent.

13. The device according to claim 12, **characterized in that** the reactor is a fixed-bed reactor.

14. The device according to claim 12, **characterized in that** the reactor is a membrane reactor.
